# EUROPÄISCHE PATENTSCHRIFT

(11) **EP 0 451 760 B1**
(45) Veröffentlichungstag und Bekanntmachung des Hinweises auf die Patenterteilung: **13.09.1995**
(21) Anmeldenummer: 91105540.8
(22) Anmeldetag: 08.04.1991
(51) Int. Cl.: C07C 323/59, C07C 327/34, A61K 31/265

(54) **Nitratoalkancarbonsäure-Derivate und Verfahren zu ihrer Herstellung**
Nitrato alkanoic acid derivatives and method for their production
Nitrato acides alkanoiques et procédé pour leur préparation

(30) Priorität: 10.04.1990 DE 4011505
(43) Veröffentlichungstag der Anmeldung: 16.10.1991
(73) Patentinhaber: SCHWARZ PHARMA AG, D-40789 Monheim (DE)
(72) Erfinder: Sandrock, Klaus, Dr., W-4018 Langenfeld (DE); Noack, Eike, Prof.Dr., W-4040 Neuss 21 (DE); Fritschi, Edgar, Dr., W-4056 Schwalmtal-Lüttelforst (DE); Kanzler, Ralf, W-5090 Leverkusen 1 (DE); Feelisch, Martin, Dr., W-4000 Düsseldorf 1 (DE)

(56) Entgegenhaltungen:
- EP-A- 0 362 575

## Beschreibung

Die Erfindung betrifft Nitratoalkancarbonsäure-Derivate, Verfahren zu deren Herstellung und diese enthaltende Arzneimittel.

Organische Nitrate (Salpetersäureester) haben sich in der Therapie von Herzerkrankungen bewährt.

Sie entfalten ihre Wirkung sowohl durch eine Herzentlastung über eine Senkung von Vor- und Nachlast als auch durch eine Verbesserung des Sauerstoffangebotes für das Herz über eine Erweiterung der Koronargefäße.

Allerdings hat man in den vergangenen Jahren festgestellt, daß die bisher in der Therapie eingesetzten organischen Nitrate, wie Glyceroltrinitrat (GTN), Isosorbid-5-mononitrat oder Isosorbiddinitrat bei hoher und kontinuierlicher Zufuhr zum Organismus innerhalb relativ kurzer Zeit eine deutliche Abschwächung der Wirkung, die Nitrattoleranz, aufweisen. Zahlreiche Experimente weisen darauf hin, daß die Anwesenheit von Sulfhydryl-Gruppen die Entstehung einer Nitrattoleranz verhindern und eine bereits eingetretene Toleranz abschwächen kann.

Der Mechanismus der Toleranzerzeugung wird heute so verstanden:
Nach derzeitigem Kenntnisstand ist die pharmakologische Wirkung der organischen Nitroverbindungen von der Anwesenheit von Cystein abhängig. Mit diesem bildet das organische Nitrat eine gemeinsame Vorstufe, aus deren Zerfall u.a. NO-Radikale freigesetzt werden, die das Zielenzym, die lösliche Guanylatzyklase der glatten Muskelzelle, aktiviert. Weitere, durch die Bildung von cGMP ausgelöste Folgereaktionen, führen dann zur Relaxation bzw. Gefäßdilatation.

Bei dem reaktiven und kurzlebigen, bisher noch hypothetischen Intermediärprodukt dürfte es sich um den Thioester der Salpetersäure oder ein Thionitrat handeln. Durch intramolekulare Umlagerung und weitere Folgereaktionen, die noch nicht aufgeklärt sind, wird letztlich die Bildung eines Nitrosothiols postuliert, aus dem dann Stickstoffmonoxid bzw. Nitritionen freigesetzt werden. Der enzymabhängige Abbau mit Hilfe der GSH-Reduktase dürfte dagegen, da er ausschließlich zur Bildung von Nitritionen führt, für die pharmakologische Wirkung ohne Bedeutung sein. Der nichtenzymatische Abbau benötigt also, wie geschildert, Cystein und ist damit dosisabhängig erschöpfbar (Erschöpfung des SH-Gruppen-pools), so daß auf Dauer nicht mehr genug NO als eigentlicher Aktivator der Guanylzyklase gebildet werden kann und es klinisch zu einer Wirkungsabschwächung kommt.

In der EP-A-0 362 575, : dessen Inhalt gemäß Artikel 59(3) und (4) EPÜ für die vorliegenden Anmeldung als Stand der Technik gilt, werden spezifisch aufgebaute Verbindungen genannt, die sich aus Nitratofettsäuren (Nitratoalkancarbonsäuren) und schwefelhaltigen Aminosäuren bzw. Peptiden zusammensetzen. Die Anwesenheit von Sulfhydrylgruppen sollen eine Nitrattoleranz oder eine bereits eingetretene Toleranz verhindern oder abschwächen.

Es werden u.a. Verbindungen genannt, die schwefelhaltige Aminosäuren wie Cystein oder Methionin in Form ihrer Methyl-, Ethyl- oder Propylester enthalten. Schließlich kann die SH-Gruppe des Cysteins mit Niederalkancarbonsäure mit 2 bis 8 Kohlenstoffatomen verestert sein.

Obwohl diese Verbindungen bereits wertvolle pharmakologische Eigenschaften im Hinblick auf Vermeidung einer Nitrattoleranz bzw. Verhinderung oder Abschwächung bereits eingetretener Toleranz aufweisen, sind sie mit Nachteilen behaftet. Sie besitzen niedrige Schmelzpunkte, weisen eine geringe Wasserlöslichkeit auf und bereiten Schwierigkeiten in der Reindarstellung. Weiterhin ist ihre pharmakologische Wirkstärke relativ gering.

Aufgabe der vorliegenden Erfindung ist es daher, neue organische Verbindungen herzustellen und dem Fachmann zur Verfügung zu stellen, die die o.g. Nachteile vermeiden und gesteigerte pharmakologische Wirksamkeit, insbesondere Wirkstärke, aufweisen.

Diese Aufgabe wurde dadurch gelöst, daß die erfindungsgemäßen Verbindungen Nitratoalkancarbonsäure-Derivate der allgemeinen Formel (I) sind 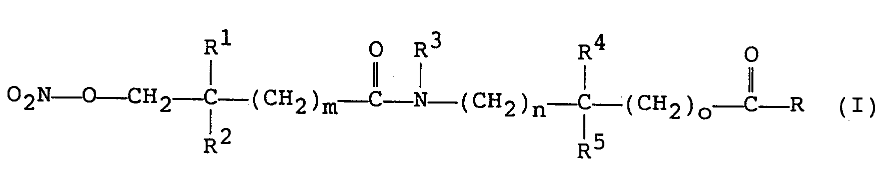
worin
- R: Hydroxy, Amino oder Äthoxy ist,
- R¹: Wasserstoff oder Alkyl mit 1 bis 6 Kohlenstoffatomen ist,
- R²: Wasserstoff oder Alkyl mit 1 bis 6 Kohlenstoffatomen ist,
- R³: Wasserstoff ist,
- R⁴: Wasserstoff, Phenyl oder Methoxyphenyl ist,
- R⁵: S-Acylderivate von Mercaptomethyl, ausgewählt von Aminosäurethioester, N-Acylaminosäurethioester, Peptidthioester oder N-Acylpeptidthioester mit 2-5 peptidverknüpften Aminosäureresten bedeutet,
- R und R⁴: miteinander unter Bildung eines Esters oder Amids verbunden sein können,
- R³ und R⁴: miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, verbunden sein können,
- m, n und o: die Zahlen 0 - 10 bedeuten
sowie deren physiologisch verträgliche Salze.

Die erfindungsgemäßen Verbindungen können auch in Form ihrer Säure-Additionssalze vorliegen.

Nach einer Weiterbildung der Erfindung besitzen die Nitratofettsäurebestandteile eine Kettenlänge von C₂ - C₆; sie können geradkettig, verzweigt, racemisch oder optisch isomer sein.

Bevorzugt enthalten die erfindungsgemäßen Nitratoalkancarbonsäure-Derivate der allgemeinen Formel (I) aus der Reihe schwefelhaltiger Aminosäuren die Aminosäuren Cystein, Methionin, Homocystein oder β,β-dimethylcystein.

Nach einer weiteren Ausbildung der Erfindung liegen die Aminosäuren in der stereochemischen L-Form vor.

Die schwefelhaltigen Aminosäuren können am C-terminalen Ende verestert sein.

Nach einer vorteilhaften erfindungsgemäßen Weiterbildung liegen die Aminosäuren Cystein, Homocystein, Methionin und/oder β,β-dimethylcystein als Methyl-, Ethyl- oder Propylester vor.

Insbesondere
N-Nitratopivaloyl-S-(N-acetylglycyl)-L-cysteinethylester,
N-Nitratopivaloyl-S-(N-acetylalanyl)-L-cysteinethylester sowie
N-Nitratopivaloyl-S-(N-acetylleucyl)-L-cysteinethylester
sind im Sinne der Erfindung bevorzugt.

Die erfindungsgemäßen Verbindungen der allgemeinen Formel (I) können in an sich bekannter Weise dadurch hergestellt werden, daß man eine Verbindung der allgemeinen Formel (II) 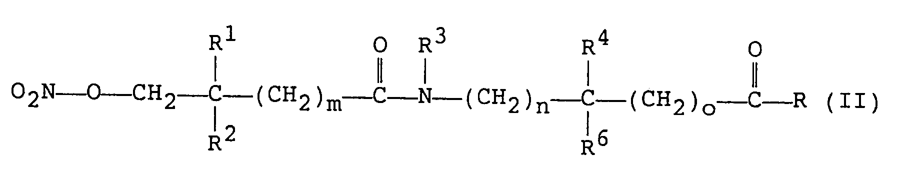
in der
- R: Hydroxy, Amino oder Äthoxy ist,
- R¹: Wasserstoff oder Alkyl mit 1 bis 6 Kohlenstoffatomen ist,
- R²: Wasserstoff oder Alkyl mit 1 bis 6 Kohlenstoffatomen ist,
- R³: Wasserstoff ist,
- R⁴: Wasserstoff, Phenyl oder Methoxyphenyl ist,
- R⁶: Mercaptomethyl ist,
- R und R⁴: miteinander unter Bildung eines Esters oder Amids verbunden sein können,
- R³ und R⁴: miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, verbunden sein können,
einer an sich bekannten Thioester-Bildungsreaktion mit Aminosäuren, N-Acylaminosäuren, Peptiden oder N-Acylpeptiden mit 2-5 peptidverknüpften Aminosäureresten unterwirft, wobei N-Acetylglycin, N-Acetylalanin oder N-Acetylleucin bevorzugt sind. Die Herstellung einer Verbindung der allgemeinen Formel (II) kann in der Weise erfolgen, wie sie in EP 89 116 700.9 beschrieben wurde.

Danach können Nitratofettsäuren der allgemeinen Formel (A), 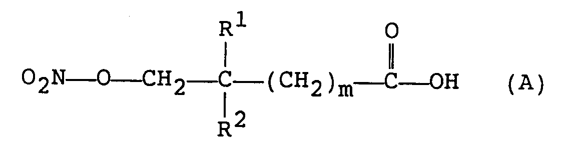
in der R¹, R² und m die oben genannten Bedeutungen haben, in Form ihrer freien Säuren, reaktiven Säurehalogenide, Säure-Azide, Ester und Säure-Anhydride eingesetzt werden und mit einer Verbindung der allgemeinen Formel (B) 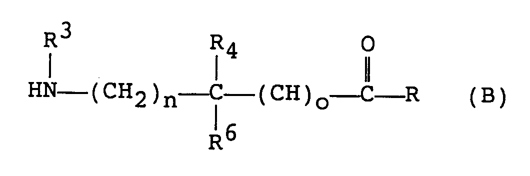
in der Bedeutung für R, R³, R⁴, R⁶, n und o wie oben genannt, enthaltend Aminosäuren und/oder Peptide unter Bildung von Verbindungen der allgemeinen Formel (II) umgesetzt werden.

Reaktive Derivate der Nitratoalkancarbonsäuren, die verfahrensgemäß eingesetzt werden können, sind beispielsweise: Säurehalogenide, Säureanhydride, aktivierte Amide und aktivierte Ester. Bevorzugt sind Säurechloride, Säureazide, symmetrische Säureanhydride, aktivierte Ester und gemischte Anhydride mit organischen oder anorganischen Säuren.

Die Kondensationsreaktion einer Nitratoalkancarbonsäure mit einer Aminogruppe einer Aminosäure kann auch in einem inerten Lösungsmittel und in Anwesenheit eines kondensationsfördernden Mittels, das die Ausbildung der Säureamidbindung fördert, wie einem Carbodiimid, N,N'-dicyclohexylcarbodiimid oder ähnlichen Carbodiimiden, einer Iminverbindung, wie Diphenylketen-N-cyclohexylimin oder Pentamethylen-Keten-N-cyclohexylimin oder einem Phosphat oder Phosphit wie Triethylphosphit, Ethylpolyphosphat oder Isopropylpolyphosphat innerhalb 1 - 48 Std. bei Temperaturen von -10°C bis zur Rückflußtemperatur des Lösungsmittels ausgeführt werden.

Aminosäuren, die verfahrensgemäß eingesetzt werden können, sind Glycin, N-Acetylglycin, Alanin, N-Acetylalanin, Arginin, N-Acetylarginin, N-α-Benzoylarginin, Cystein, N-Acetylcystein, N,S-Dipivaloylcystein, Cystein, N,N-Diacetylcystein, Leucin, N-Acetylleucin, Lysin, N-α-Acetyllysin, N- -Acetyllysin, N-α-Diacetyllysin, Prolin, N-Acetylprolin, Serin, N-Acetylserin, O-Acetylserin, N,O-Diacetylserin, Methionin, N-Benzoylmethionin, Phenylalanin, N-Benzoylphenylalanin, N-Acetylphenylalanin, Asparagin, N-Acetylasparagin, N-Acetylasparagin-monoethylester, Glutaminsäure und N-Acetylglutaminsäuremonomethylester.

Zur Überführung der Verbindungen der allgemeinen Formel (I) in ihre pharmakologisch unbedenklichen Salze setzt man diese, vorzugsweise in einem organischen oder wässrig organischem Lösungsmittel, mit der äquivalenten Menge einer gegebenenfalls anorganischen oder organischen Säure um. In Betracht kommen Salzsäure, Bromwasserstoffsäure, Salpetersäure, Phosphorsäure, Schwefelsäure, Ameisensäure, Essigsäure, Propionsäure, Oxalsäure, Fumarsäure, Maleinsäure, Bernsteinsäure, Adipinsäure, Benzoesäure, Salicylsäure, O-Acetoxybenzoesäure, Zimtsäure, Naphtoesäure, Mandelsäure, Citronensäure, Äpfelsäure, Weinsäure, Asparaginsäure, Glutaminsäure, Methansulfonsäure oder p-Toluolsulfonsäure.

Die erfindungsgemäßen neuen Verbindungen der allgemeinen Formel (I) und ihre Salze können in flüssiger oder fester Form oral, enteral oder parenteral appliziert werden.

Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Ethanol, Komplexbildner (wie Ethylendiamintetraessigsäure und deren nicht toxische Salze), hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselgelsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie z.B. Polyethylenglykole); für orale Applikation geeignete Zubereitungen können gewünschtenfalls Geschmacks- und Süßstoffe enthalten.

Nach einer weiteren Ausbildung der Erfindung weisen Arzneimittel einen Gehalt an einer und/oder einem Gemisch der erfindungsgemäßen Verbindungen auf.

Diese Arzneimittel können zur Behandlung von Kreislauferkrankungen, beispielsweise als Coronardilatatoren, als Mittel zur Behandlung von Hochdruck, Herzinsuffizienz und zur Erweiterung der peripheren Gefäße, einschließlich der Hirn- und Nierengefäße eingesetzt werden.

Pharmazeutische Zubereitungen, eine vorausberechnete Menge einer oder mehrerer der erfindungsgemäßen Verbindungen enthaltend, können einmal täglich in Form retardierter Zubereitungen oder mehrmals täglich in regelmäßigen Intervallen (2 - 3 mal täglich) verabreicht werden. Die üblicherweise pro Tag applizierten Wirkstoffmengen sind 20 - 300 mg pro Tag, bezogen auf 75 kg Körpergewicht. In Form von Injektionen können die erfindungsgemäßen Verbindungen 1 - 8 mal pro Tag bzw. durch Dauerinfusion gegeben werden. Normalerweise reichen Mengen von 5 - 200 mg/Tag aus.

Eine typische Tablette kann folgende Zusammensetzung aufweisen:

| | |
|---|---|
| 1) N-Nitratopivaloyl-S-(N-acetylglycyl)-L-cysteinethylester | 25 mg |
| 2) Stärke, U.S.P. | 57 mg |
| 3) Lactose, U.S.P. | 73 mg |
| 4) Talkum, U.S.P. | 9 mg |
| 5) Stearinsäure | 6 mg |

Die Stoffe 1, 2 und 3 werden gesiebt, granuliert, mit 4 und 5 homogen gemischt und anschließend tablettiert.

Die Ausführungsbeispiele, ohne darauf beschränkt zu sein, erläutern die Erfindung.

### Ausführungsbeispiel 1

### Darstellung von N-Nitratopivaloyl-S-(N-acetylglycyl)-L-cysteinethylester

48 g (0,41 mol) N-Acetylglycin wurden unter Rühren bei Raumtemperatur in 300 ml Methylenchlorid (CH₂Cl₂) aufgeschlämmt und auf 10°C gekühlt. Unter Rühren wurde eine Lösung von 109,8 g (0,373 mol) N-Nitratopivaloyl-L-cysteinethylester in 300 ml CH₂Cl₂ bei schwach exothermer Reaktion zugegeben. Das Reaktionsgemisch wurde unter Rühren auf 5°C gekühlt und langsam eine Lösung von 84,6 g (0,41 mol) Dicyclohexylcarbodiimid (DCC) in 200 ml CH₂Cl₂ unter Rühren zugetropft, daß die Temperatur im Bereich zwischen 5°C und 10°C lag. Nach Erwärmung auf Raumtemperatur wurde vier Tage bei RT gerührt. DCC-Harnstoff wurde abgesaugt und zweimal mit je 100 ml CH₂Cl₂ gewaschen.

Die vereinigten CH₂Cl₂ - Phasen wurden nacheinander jeweils einmal mit 200 ml 9 %iger NaHC0₃-Lösung, 300 ml 1 n HCl und 300 ml destilliertem Wasser gewaschen. Schließlich wurde die Methylenchlorid-Phase über wasserfreiem Natriumsulfat getrocknet und am Rotationsverdampfer (Rotavapor®, Büchi) bis zur Gewichtskonstanz eingeengt.

Die Ausbeute betrug 162,9 g (theoretisch 146,74 g) N-Nitratopivaloyl-S-(N-acetylglycyl)-L-cysteinethylester als hellgelbes Öl.

162,9 g N-Nitratopivaloyl-S-(N-acetylglycyl)-L-cysteinethylester wurden in 470 ml Ethylacetat bei Raumtemperatur gelöst. Nach 15-minütigem Rühren bei RT wurde ungelöster weißer Niederschlag abfiltriert. Das klare hellgelbe Filtrat wurde bei RT unter Rühren langsam mit 390 ml n-Hexan versetzt.

Dieser Lösung wurden Impfkristalle zugesetzt und bei Raumtemperatur über Nacht gerührt. Die ausgefallenen Kristalle wurden abgesaugt und zweimal mit je 100 ml eines Gemisches aus 20 ml Ethylacetat und 80 ml n-Hexan bei Raumtemperatur gewaschen.

Die Kristalle wurden im Vakuumtrockenschrank bei Raumtemperatur, Vakuum 267 Pa, bis zur Gewichtskonstanz getrocknet.

Die Ausbeute betrug 85,4 g (theoretisch 146,74 g) N-Nitratopivaloyl-S-(N-acetylglycyl)-L-cysteinethylester. Fp: 71,8°C

### Ausführungsbeispiel 2

### Darstellung von N-Nitratopivaloyl-S-(N-acetylalanyl)-L-cysteinethylester

53,8 g (0,41 mol) N-Acetylalanin wurden unter Rühren bei Raumtemperatur in 300 ml Methylenchlorid (CH₂Cl₂) aufgeschlämmt und auf 10°C gekühlt. Unter Rühren wurde eine Lösung von 109,8 g (0,373 mol) N-Nitratopivaloyl-L-cysteinethylester in 300 ml CH₂Cl₂ bei schwach exothermer Reaktion zugegeben. Das Reaktionsgemisch wurde unter Rühren auf 5°C gekühlt und langsam eine Lösung von 84,6 g (0,41 mol) Dicyclohexylcarbodiimid (DCC) in 200 ml CH₂Cl₂ unter Rühren zugetropft, daß die Temperatur im Bereich zwischen 5°C und 10°C lag. Nach Erwärmung auf Raumtemperatur wurde vier Tage bei RT gerührt. DCC-Harnstoff wurde abgesaugt und zweimal mit je 100 ml CH₂Cl₂ gewaschen.

Die vereinigten Methylenchlorid-Phasen wurden nacheinander jeweils einmal mit 200 ml 9 %iger NaHC0₃-Lösung, 300 ml 1 n HCl und 300 ml destilliertem Wasser gewaschen. Schließlich wurde die Methylenchlorid-Phase über wasserfreiem Natriumsulfat getrocknet und am Rotatationsverdampfer (Rotavapor®, Büchi) bis zur Gewichtskonstanz eingeengt.

Die Ausbeute betrug 160,5 g (theoretisch 151,84 g) N-Nitratopivaloyal-S-(N-acetylalanyl)-L-cysteinethylester als hellgelbes Öl. 160,5 g N-Nitratopivaloyl-S-(N-acetylalanyl)-L-cysteinethylester wurden in 345 ml Ethylacetat bei Raumtemperatur gelöst. Nach 15-minütigem Rühren bei RT wurde ungelöster weißer Niederschlag abfiltriert. Das klare hellgelbe Filtrat wurde bei RT unter Rühren langsam mit 345 ml n-Hexan versetzt.

Dieser Lösung wurden Impfkristalle zugesetzt und bei Raumtemperatur über Nacht gerührt. Die ausgefallenen Kristalle wurden abgesaugt und zweimal mit je 100 ml eines Gemisches aus 20 ml Ethylacetat und 80 ml n-Hexan bei Raumtemperatur gewaschen.

Die Kristalle wurden im Vakuumtrockenschrank bei Raumtemperatur, Vakuum 267 Pa, bis zur Gewichtskonstanz getrocknet.

Die Ausbeute betrug 78,2 g (theoretisch 151,84 g) N-Nitratopivaloyl-S-(N-acetylalanyl)-L-cysteinethylester. Fp: 76,6°C

### Ausführungsbeispiel 3

### Darstellung von N-Nitratopivaloyl-S-(N-acetylleucyl)-L-cysteinethylester

0,02 Mol ≙ 6 g Nitratopivalinsäurecysteinethylester werden in 100 ml Dichlormethan gelöst. Bei 10°C und Stickstoff-Zufuhr werden langsam 0,03 Mol ≙ 5,19 g N-Acetyl-Leucin und 0,1 g Dimethylaminopyridin (DMAP) zugegeben. Anschließend werden 0,03 Mol ≙ 6,15 g Dicyclohexylcarbodiimid (DCC), gelöst in 80 ml Dichlormethan, zugetropft. Es wird über Nacht bei RT gerührt.

### Aufarbeitung:

Der Ansatz wird abgesaugt. Die Lösung wird nacheinander mit äquivalenten Mengen 0,1 n HCl-Lösung, gesättigter NaHCO₃-Lösung und H₂0 dest. extrahiert. Anschließend wird das Lösungsmittel am Rotationsverdampfer (Rotavapor® , Büchi) abgezogen. Es bleiben 10 g öliger Rückstand zurück.

### Umkristallisation:

Es werden 10 g ölige Substanz unter leichtem Erwärmen in 45 ml Ethanol und 40 ml H₂0 dest. gelöst. Über Nacht läßt man die Substanz im Kühlschrank auskristallisieren. Die Kristalle werden abgesaugt und im Vakuumtrockenschrank getrocknet.

Massenspektrum bestätigt Struktur.

| | |
|---|---|
| Schmelzpunkt: | 91,4°C |
| HPLC-Analytik: | 98,7 % |
| Ausbeute: | 5 g = 0,012 Mol = 57,4 % d. Th. |

### Pharmakologische Daten

Das hämodynamische Profil der beispielhaft ausgewählten erfindungsgemäßen Verbindungen N-Nitratopivaloyl-S-(N-acetyl-glycyl)-L-cysteinethylester und N-Nitratopivaloyl-S-(N-acetylalanyl)-L-cysteinethylester wurde im direkten Vergleich zu klassischen Nitraten am wachen Hund (Beagle dogs) erforscht. Die perorale wie auch die intravenöse Applikation beider o.g. Verbindungen (Dosisbereich: 0,01 bis 1 µmol/kg i.v. bzw. 1 bis 10 µmol/kg p.o.) führt zu einer dosisabhängigen Abnahme im mittleren arteriellen und venösen Druck mit prädominanter Wirkung an den Gefäßen. Die arterielle Gefäßausdehnung war gesteigert, während die Herzleistung unverändert blieb. Die bestimmte Wirkdauer charakterisierte beide Verbindungen als langwirkende Nitrate.

Die ermittelten Dosis-Wirkungs-Kurven für die o.g. Verbindungen, GTN (Glyceroltrinitrat) und IS-5-N (Isosorbid-5-mononitrat) zeigten deutlich, daß beide neuen Nitratverbindungen in vivo effektiver sind als das wirksamste klassische Nitrat bezüglich der Verminderung des arteriellen und zentral venösen Blutdruckes.

Die folgenden Daten zeigen die Wirkung einer i.v.-Applikation von 1,7 µmol/kg zweier ausgewählter erfindungsgemäßer Verbindungen beim systolischen arteriellen und zentral venösen Blutdruck an wachen Beagle-Hunden (n = 15).

| Verbindung | △ SAPₘₐₓ | △ CVPₘₐₓ |
|---|---|---|
| N-Nitratopivaloyl-S-(N-acetylglycyl)-L-cysteinethylester | - 23 ± 3 | - 46 ± 4 |
| N-Nitratopivaloyl-S-(N-acetylalanyl)-L-cysteinethylester | - 21 ± 2 | - 46 ± 4 |
| GTN | - 25 ± 3 | - 35 ± 4 |
| IS-5-N | - 6 ± 1 | - 16 ± 6 |
| SAP = systolic arterial pressure CVP = central venous pressure | | |

## Patentansprüche (Patentansprüche für folgende(n) Vertragsstaat(en): AT, BE, CH, DE, DK, FR, GB, IT, LI, LU, NL, SE)

1. Nitratoalkancarbonsäure-Derivate der allgemeinen Formel (I) 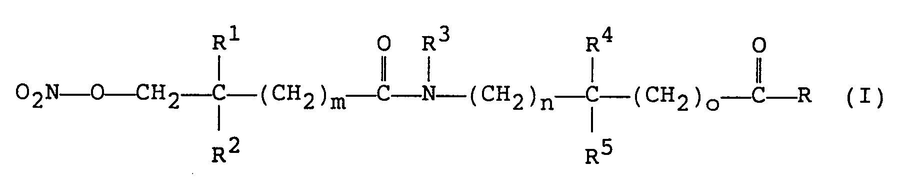 worin
R Hydroxy, Amino oder Äthoxy ist,
R¹ Wasserstoff oder Alkyl mit 1 bis 6 Kohlenstoffatomen ist,
R² Wasserstoff oder Alkyl mit 1 bis 6 Kohlenstoffatomen ist,
R³ Wasserstoff ist,
R⁴ Wasserstoff, Phenyl oder Methoxyphenyl ist,
R⁵ S-Acylderivate von Mercaptomethyl, ausgewählt von Aminosäurethioester, N-Acylaminosäurethioester, Peptidthioester und N-Acylpeptidthioester mit 2-5 peptidverknüpften Aminosäureresten bedeutet,
R und R⁴ miteinander unter Bildung eines Esters oder Amids verbunden sein können,
R³ und R⁴ miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, verbunden sein können,
m, n und o die Zahlen 0 - 10 bedeuten,
sowie deren pharmakologisch verträgliche Säureadditionssalze.

2. Verbindungen nach Anspruch 1, dadurch gekennzeichnet, daß deren Nitratofettsäurebestandteile eine Kettenlänge von C₂-C₆ haben, geradkettig, verzweigt, racemisch oder optisch isomer sind.

3. Verbindungen nach Ansprüchen 1 - 2, dadurch gekennzeichnet, daß die schwefelhaltigen Aminosäuren bevorzugt Cystein, Methionin, Homocystein oder β,β-Dimethylcystein sind.

4. Verbindungen nach Ansprüchen 1 - 3, dadurch gekennzeichnet, daß die Aminosäuren in der stereochemischen L-Form vorliegen.

5. Verbindungen nach Ansprüchen 1 - 4, dadurch gekennzeichnet, daß die schwefelhaltigen Aminosäuren am C-terminalen Ende verestert sind.

6. Verbindungen nach Ansprüchen 1 - 5, dadurch gekennzeichnet, daß die Aminosäuren Cystein, Homocystein, β,β-Dimethylcystein und/oder Methionin als Methyl-, Ethyl- oder Propylester vorliegen.

7. Verbindungen, die die folgenden chemischen Formeln aufweisen:
a) N-Nitratopivaloyl-S-(N-acetylglycyl)-L-cysteinethylester
b) N-Nitratopivaloyl-S(N-acetylalanyl)-L-cysteinethylester
c) N-Nitratopivaloyl-S-(N-acetylleucyl)-L-cysteinethylester

8. Verfahren zur Herstellung der Nitratoalkancarbonsäure-Derivate nach Anspruch 1, dadurch gekennzeichnet, daß man jeweils in an sich bekannter Weise
a) eine Verbindung der allgemeinen Formel (A) 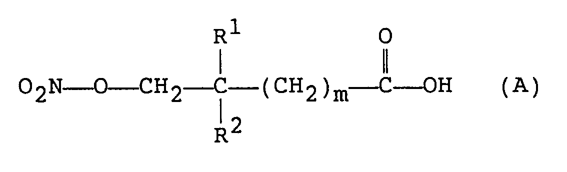 in Form ihrer freien Säure, ihrer reaktiven Säurehalogenide, Säureazide, Ester oder Säureanhydride mit der Aminogruppe einer Verbindung der allgemeinen Formel (B) 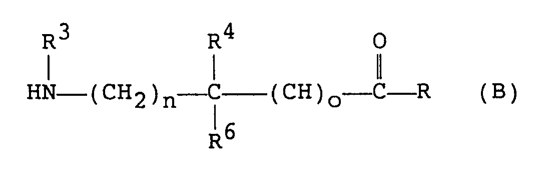 worin jeweils R, R¹, R², R³ und R⁴ die in Anspruch 1 angegebenen Bedeutungen haben und R⁶ für -CH₂-SH steht, zu Verbindungen der allgemeinen Formel (II) 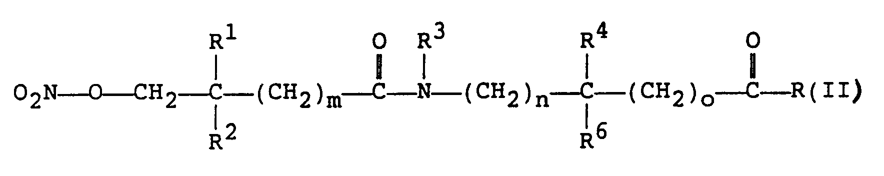 umsetzt und
b) die Verbindungen (II) anschließend mit N-Acetylglycin, N-Acetylalanin, N-Acetylleucin oder N-Acetylpeptiden mit 2 bis 5 peptidverknüpften Aminosäuren reagieren läßt und danach die erhaltenen Verbindungen gegebenenfalls in ihre pharmakologisch verträglichen Salze überführt.

9. Arzneimittel enthaltend eine oder mehrere Verbindung(en) gemäß Ansprüchen 1 - 7 sowie übliche Träger- und Hilfsstoffe.

10. Verwendung der Verbindungen gemäß Ansprüchen 1 - 7 zur Herstellung von Arzneimitteln zur Prophylaxe von Herz- und Kreislauferkrankungen.

## Patentansprüche (Patentansprüche für folgende(n) Vertragsstaat(en): ES, GR)

1. Verfahren zur Herstellung von Nitratoalkancarbonsäure-Derivaten sowie deren pharmazeutisch akzeptablen Salze der allgemeinen Formel (I) 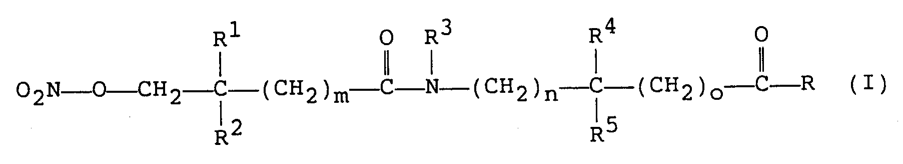 dadurch gekennzeichnet, daß eine Verbindung entsprechender Nitratofettsäuren der allgemeinen Formel (A) 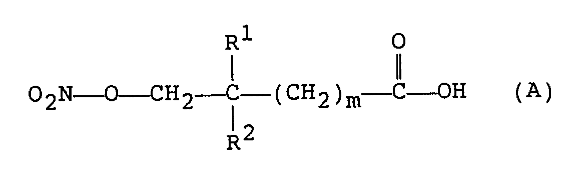 in Form ihrer freien Säure, ihrer reaktiven Säurehalogenide, Säureazide, Ester oder Säureanhydride mit der Aminogruppe einer Verbindung der allgemeinen Formel (B) 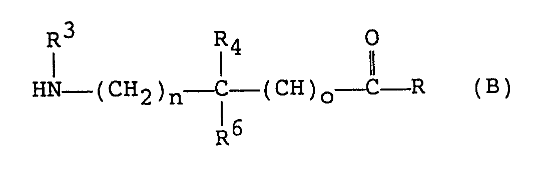 kondensiert wird, um eine Verbindung der allgemeinen Formel (II) zu bilden, 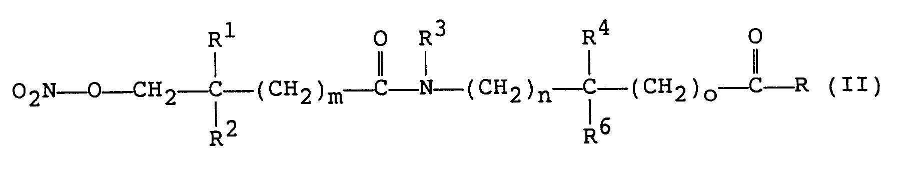 wobei in den allgemeinen Formeln (A), (B) (I) und (II) bedeutet:
R = Hydroxy, Amino oder Äthoxy;
R¹ = Wasserstoff oder Alkyl mit 1 bis 6 Kohlenstoffatomen;
R² = Wasserstoff oder Alkyl mit 1 bis 6 Kohlenstoffatomen;
R³ = Wasserstoff;
R⁴ = Wasserstoff, Phenyl oder Methoxyphenyl;
R⁵ = S-Acylderivate von Mercaptomethyl, ausgewählt von Aminosäurethioster, N-Acylaminosäurethioester, Peptidthioester und N-Acylpeptidthioester mit 2-5 peptidverknüpften Aminosäureresten,
R⁶ = Mercaptomethyl,
R und R⁴ miteinander unter Bildung eines Esters oder Amids verbunden sein können,
R³ und R⁴ miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, verbunden sein können,
m, n und o die Zahlen 0 - 10 bedeuten,
und man die jeweiligen Verbindungen (II) anschließend mit N-Acetylglycin, N-Acetylalanin, N-Acetylleucin oder N-Acetylpeptiden mit 2 bis 5 peptidverknüpften Aminosäuren reagieren läßt und danach die erhaltenen Verbindungen gegebenenfalls in ihre pharmakologisch verträglichen Salze überführt.

2. Verfahren nach Anspruch 1, dadurch gekennzeichnet, daß Nitratofettsäuren mit einer Kettenlänge von C₂-C₆, die linear oder verzweigt, racemisch oder optisch isomer sein können, verwendet werden.

3. Verfahren nach Ansprüchen 1 und 2, dadurch gekennzeichnet, daß als schwefelhaltige Aminosäuren bevorzugt Cystein, Methionin, Homocystein oder β,β-Dimethylcystein verwendet werden.

4. Verfahren nach einem der Ansprüche 1 - 3, dadurch gekennzeichnet, daß als Aminosäuren Säuren in der stereochemischen L-Form verwendet werden.

5. Verfahren nach einem der Ansprüche 1 - 4, dadurch gekennzeichnet, daß die schwefelhaltigen Aminosäuren, am C-terminalen Ende verestert, verwendet werden.

6. Verfahren nach einem der Ansprüche 1 - 5, dadurch gekennzeichnet, daß die Aminosäuren Cystein, Homocystein, β, β-Dimethylcystein und/oder Methionin in Form ihrer Methyl-, Ethyl- oder Propylester vorliegend, verwendet werden.

7. Verfahren zur Herstellung von Nitratoalkancarbonsäure-Derivaten sowie deren pharmazeutisch akzeptablen Salze nach Anspruch 1, dadurch gekennzeichnet, daß die Verbindungen die folgenden chemischen Bezeichnungen aufweisen:
a) N-Nitratopivaloyl-S-(N-acetylglycyl)-L-cysteinethylester
b) N-Nitratopivaloyl-S-(N-acetylalanyl)-L-cysteinethylester
c) N-Nitratopivaloyl-S-(N-acetylleucyl)-L-cysteinethylester

8. Verfahren zur Herstellung von Arzneimitteln, dadurch gekennzeichnet, daß eine Verbindung oder ein Gemisch von Verbindungen hergestellt gemäß Ansprüchen 1 - 7 und/oder deren Derivate und/oder deren pharmazeutisch annehmbaren Salze gegebenenfalls mit pharmazeutischen Hilfsstoffen in eine Arzneiform verbracht werden.

## Claims (Claims for the following Contracting State(s): AT, BE, CH, DE, DK, FR, GB, IT, LI, LU, NL, SE)

1. Nitrato alkanoic acid derivatives of the general formula (I) 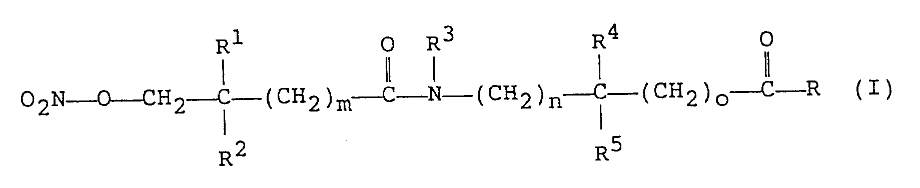 wherein
R represents hydroxy, amino or ethoxy;
R¹ represents hydrogen or alkyl with 1 to 6 carbon atoms;
R² represents hydrogen or alkyl with 1 to 6 carbon atoms;
R³ represents hydrogen;
R⁴ represents hydrogen, phenyl or methoxyphenyl;
R⁵ represents S-acyl derivatives of mercaptomethyl, chosen from amino-acid thio esters, N-acyl-amino thio esters, peptide thio esters or N-acylpeptide thio esters with 2-5 peptide bonded amino acid groups;
groups in which R and R⁴ are bonded together in the form of an ester or an amide,
groups in which R³ and R⁴ are bonded together in the form of an alkylene bridge with 2 to 4 carbon atoms, an alkylene bridge with 2 to 3 carbon atoms and a sulfur atom, an alkylene bridge with 3 to 4 carbon atoms, which contains a double bond or an alkylene bridge as mentioned above which can be substituted with a member of the group consisting of hydroxy;
m, n and o are whole numbers from 0 to 10, and pharmacologically acceptable salts thereof.

2. Compounds according to claim 1 characterized in that the nitrato alkanoic acid components have a chain length of C₂ - C₆, and are selected from the group consisting of straight chain, branched chain, racemic or optical isomers.

3. Compounds according to claims 1 to 2 characterized in that the sulfur containing amino acids preferably are cysteine,methionine,homocysteine or β,β-dimethylcysteine.

4. Compounds according to claims 1 to 3 characterized in that the amino acids are in the stereochemical L-form.

5. Compounds according to claims 1 to 4 characterized in that, the sulfur containing amino acids are present on the C-terminals as esters.

6. Compounds according to claims 1 to 5 characterized in that the amino acids selected from the group consisting of cysteine, homocysteine, β,β-dimethylcysteine and/or methionine are present as an ester selected from the group consisting of methyl-, ethyl- and propylesters.

7. Compounds according to the following chemical formulas:
a) N-nitrato-pivaloyl-S-(N-acetyl-glycyl)-L-cysteine ethyl ester
b) N-nitrato-pivaloyl-S-(N-acetyl-alanyl)-L-cysteine ethyl ester
c) N-nitrato-pivaloyl-S-(N-acetyl-leucyl)-L-cysteine ethyl ester

8. Process for the manufacturing of nitratoalkanoic acid derivatives according to claim 1 characterized in that in a known manner
a) a compound of the general formula (A) 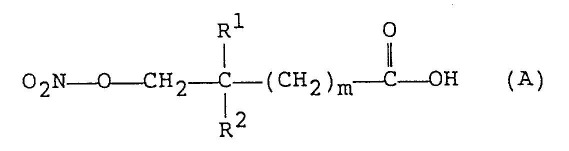 in the form of their free acid, a reactive acylhalide an acid azide, an ester or an acid anhydride is reacted with an amino group of a compound of the general formula (B) 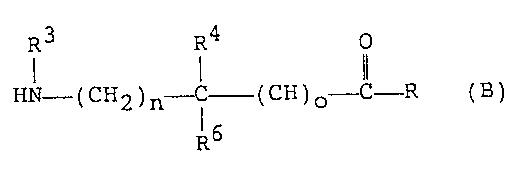 wherein respectively R, R¹, R², R³ and R⁴ have the meaning previously set forth in claim 1 and R⁶ stands for the group -CH₂ - SH thereby forming compounds of the general formula (II): 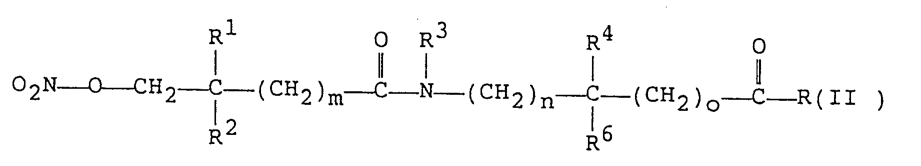 and
b) the compounds (II) subsequently are reacted with N- acetylglycine, N-acetylalanine, N-acetylleucine or N-acetylpeptides with 2 to 5 peptide bonded amino acid residues and, if desired, in form as pharmacologically acceptable salts thereof.

9. Medicaments comprising as an active ingredient a compound or a mixture of compounds according claims 1 to 7 and usual carriers and additives.

10. The use of compounds according claims 1 to 7 for the manufacture of medicaments for the prophylaxis of cardiac diseases and circulatory diseases.

## Claims (Claims for the following Contracting State(s): ES, GR)

1. Process for the manufacturing of nitrato alkanoic acid derivatives and pharmaceutically acceptable salts thereof of the general formula (I): 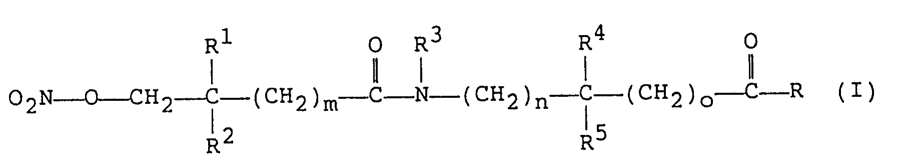 characterized in that, a compound of respective nitrato alcanoic acids of the general formula (A) 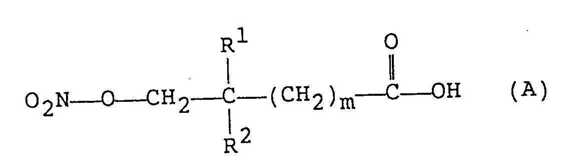 in the form of a member of the group consisting of the free acid, a reactive halide, aside, ester and/or anhydride, is reacting with the amino group of a compound of the general formula (B) 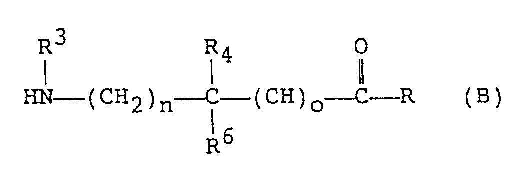 to produce a compound of the general formula (II): 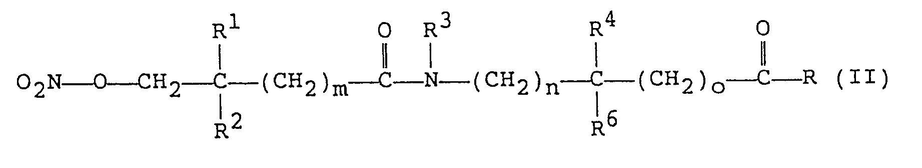 and wherein in the general formulas (A), (B), (I) and (II) represent:
R hydroxy, amino or ethoxy;
R¹ hydrogen or alkyl with 1 to 6 carbon atoms;
R² hydrogen or alkyl with 1 to 6 carbon atoms;
R³ hydrogen;
R⁴ hydrogen, phenyl or methoxyphenyl;
R⁵ S-acylderivatives of mercaptomethyl, chosen from amino acid thio esters, N-acyl-amino acid thio esters, peptide-thio esters and N-acylpeptide thio esters with 2-5 peptide bonded amino acid groups;
R⁶ mercaptomethyl;
groups in which R and R⁴ are bonded together in the form of an ester or an amide,
groups in which R³ and R⁴ are bonded together in the form of an alkylene bridge with 2 to 4 carbon atoms, an alkylene bridge with 2 to 3 carbon atoms and a sulfur atom, an alkylene bridge with 3 to 4 carbon atoms, which contains a double bond or an alkylene bridge as mentioned above which can be substituted with a member of the group consisting of hydroxy,
m, n and o are whole numbers from 0 to 10
and the respective compounds (II) subsequently are reacted with N-acetylglycine, N-acetylalanine, N-acetylleucine or N-acetylpeptides with 2 to 5 peptide bonded amino acid residues and, if desired, in form as pharmacologically acceptable salts thereof.

2. Process for the manufacturing according claim 1, characterized in that are used nitrato alkanoic acids which have a chain length of C₂ - C₆ and are selected from the group consisting of straight-chain, branched chain, racemic or optical isomers.

3. Process for the manufacturing according to claims 1 to 2, characterized in that are used preferably as sulfur containing amino acids cysteine, methionine, homocysteine or β,β-dimethylcysteine.

4. Process for the manufacturing according to claims 1 to 3 characterized in that are used amino acids in the stereochemical L-form.

5. Process for the manufacturing according to claims 1 to 4 characterized in that are used the sulfur containing amino acids which are esterified on the C-terminal.

6. Process for the manufacturing according to claims 1 to 5, characterized in that are used amino acids selected from the group consisting of cysteine, homocysteine, β,β-dimethylcysteine and/or methionine as an ester selected from the group consisting of methyl, ethyl and propyl esters.

7. Process for the manufacturing of nitrato alkanoic acid derivatives and pharmaceutically acceptable salts thereof according to claim 1, characterized in that the synthesized compounds are indicated as follows:
a) N-nitrato-pivaloyl-S-(N-acetyl-glycyl)-L-cysteine ethyl ester,
b) N-nitrato-pivaloyl-S-(N-acetyl-alanyl)-L-cysteine ethyl ester,
c) N-nitrato-pivaloyl-S-(N-acetyl-leucyl)-L-cysteine ethyl ester.

8. Process for the manufacturing of medicines, characterized in that as an active ingredient a compound or a mixture of compounds is produced according claims 1 to 7 and/or derivatives and/or pharmaceutically acceptable salts thereof comprising pharmaceutical carriers are put in a pharmaceutical form.

## Revendications (Revendications pour l'(les) Etat(s) contractant(s) suivant(s): AT, BE, CH, DE, DK, FR, GB, IT, LI, LU, NL, SE)

1. Dérivés des nitrato acides alkanoiques de la formule générale (I) 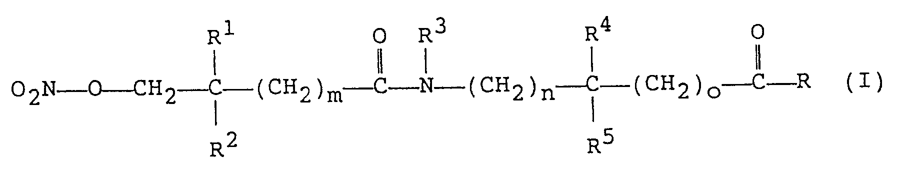 dans laquelle
R représente un radical hydroxyle, amino ou éthoxy,
R¹ représente un atome d'hydrogène ou un radical alkyle qui comporte de 1 à 6 atomes de carbone,
R² représente un atome d'hydrogène ou un radical alkyle qui comporte de 1 à 6 atomes de carbone,
R³ représente un atome d'hydrogène,
R⁴ représente un atome d'hydrogène, le radical phényle ou méthoxyphényle,
R⁵ représente des dérivés du type S-acyle du radical mercaptométhyle sélectionnés des thioester d'aminoacide, thioester d'aminoacide-N-acyle, thioester du peptide et thioester du peptide-N-acyle qui comporte de 2 à 5 radicaux d'aminoacides liés au peptide,
R et R⁴ peuvent être mutuellement liés sous formation d'un ester ou d'un amide,
R³ et R⁴ peuvent être mutuellement liés sous formation d'un pont alkylène comportant de 2 à 4 atomes de carbone, d'un pont alkylène comportant de 2 ou 3 atomes de carbon et un atome de soufre, d'un pont alkylène comportant de 3 ou 4 atomes de carbone, qui, comme plus haut, contient une double liaison ou un pont alkylène, hydroxysubstitué,
m, n et o ont des valeurs numériques qui varient de 0 à 10
et leurs sels pharmaceutiquement acceptables.

2. Composés suivant la revendication 1, caractérisés en ce que leurs composants d'acide nitratogras possèdent une longueur de chaîne de C₂ à C₆ et qu'ils sont à chaîne linéaire, à chaîne ramifiée, racémiques ou des isomères optiques.

3. Composés suivant les revendications 1 et 2,caractérisés en ce que les aminoacides contenant de soufre sont, de préférence, la cystéine, la méthionine, l'homocystéine ou la β,β-diméthylcysteine.

4. Composés suivant les revendications 1 à 3, caractérisés en ce que les aminoacides se présentent sous la forme stéréochimique L.

5. Composés suivant les revendications 1 à 4, caractérisés en ce que les aminoacides contenant de soufre sont esterfiés au bout C-terminal.

6. Composés suivant les revendications 1 à 5, caractérisés en ce que les aminoacides cystéine, homocystéine, β, β-diméthylcysteine et/ou méthionine se présentent sous forme des esters méthylique, éthylique ou propylique.

7. Composés qui possèdent les formules chimiques suivantes:
a) ester éthylique de la N-nitratopivaloyl-S-(N-acetylglycyl)-L-cysteine
b) ester éthylique de la N-nitratopivaloyl-S-(N-acetylalanyl)-L-cysteine.
c) ester éthylique de la N-nitratopivaloyl-S-(N-acetylleucyl)-L-cysteine.

8. Procédé de préparation des dérivés des nitratoacides alkanoiques suivant revendication 1, caractérisé en ce que l'on
a) fait réagir un composé de la formule générale (A) d'une manière en soi connue 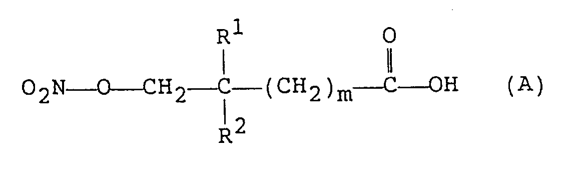 sous forme de leur acide libre, de leurs halogénures d'acides réactifs, de leurs azides, esters ou anhydrides avec le radical amino d'un composé de la formule générale (B) 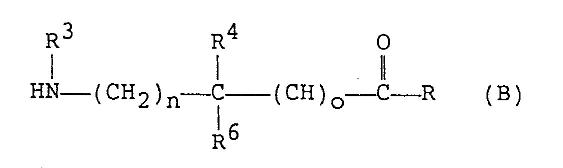 dans laquelle R, R¹, R², R³ et R⁴, chacun pour soi, a le sens selon revendication 1 et R⁶ représente -CH₂-SH de manière à engendrer un composé de la formule générale (II) et 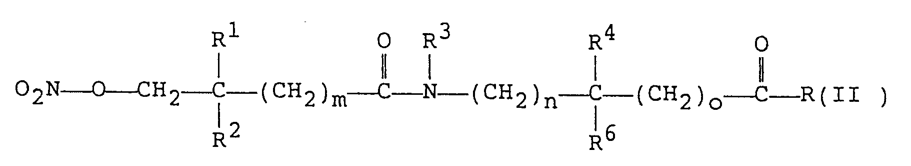
b) en ce que l'on transforme ensuite les composé (II) avec la N-acetylglycine, la N-acetylalanine, la N acetylleucine ou N-acetylpeptides qui comportent de 2 à 5 aminoacides, liés peptidiques, et éventuellement à l'aide d'une étape réactionnelle on transforme les composés obtenus en leurs sels pharmaceutiquement acceptables.

9. Médicaments qui se caractérisent par une teneur en un composé ou un mélange de composés suivant revendicatiions 1 à 7 et des adjuvants et véhicules pharmaceutiques.

10. Utilisation des composés suivant revendications 1 à 7 à préparer des médicaments qui servent au traitement de maladies du coeur et de maladies du système circulatoire.

## Revendications (Revendications pour l'(les) Etat(s) contractant(s) suivant(s): ES, GR)

1. Procédé de préparation de dérivés des nitrato acides alkanoiques ainsi que de leurs sels pharmaceutiquement acceptables de la formule générale (I) 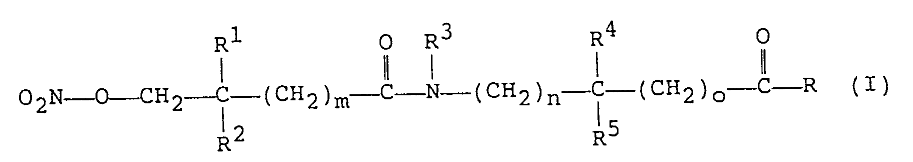 caractérisé en ce que l'on condense un composé d'acides nitrato gras correspondants de la formule générale (A) 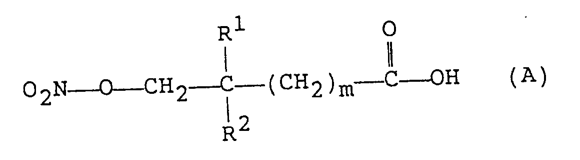 sous forme de leurs acides libres,de leurs halogenures d'acides réactifs, de leurs azides, esters ou anhydrides d'acides réactifs, avec le radical amino d'un composé de la formule générale (B) 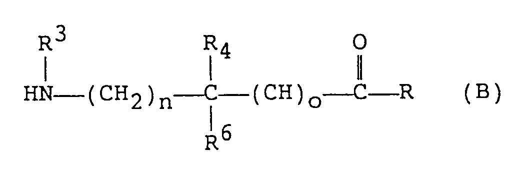 de manière à engendrer un composé de la formule générale (II) 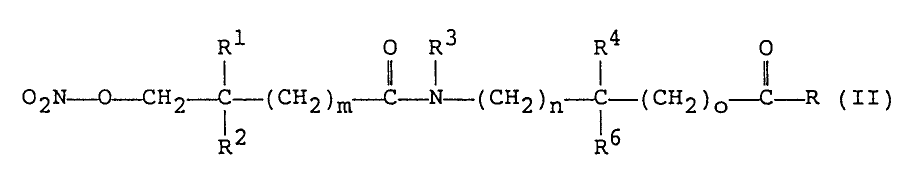 où dans les formules générales (A), (B), (I) et (II):
R représente un radical hydroxyle, amino ou ethoxy,
R¹ représente un atome d'hydrogène, un radical alkyle qui comporte de 1 à 6 atomes de carbone,
R² représente un atome d'hydrogène ou un radical alkyle qui comporte de 1 à 6 atomes de carbone,
R³ représente un atome d'hydrogène,
R⁴ représente un atome d'hydrogène,le radical phényle ou méthoxyphényle,
R⁵ représente des dérivés du type S-acyle du radical mercaptométhyle sélectionnés des thioester d'aminoacide, thioester d'aminoacide-N-acyle, thioester du peptide et thioester du peptide-N-acyle qui comporte de 2 à 5 radicaux d'aminoacides liés au peptide,
R⁶ représente mercaptométhyle,
R et R⁴ peuvent être mutuellement liés sous formation d'un ester ou d'un amide,
R³ et R⁴ peuvent être mutuellement liés sous formation d'un pont alkylène comportant de 2 à 4 atomes de carbone,d'un pont alkylène comportant de 2 ou 3 atomes de carbone et un atome de soufre, d'un pont alkylène comportant de 3 ou 4 atomes de carbone, qui, comme plus haut contient une double liaison ou un pont alkylène, hydroxysubstitué,
m, n et o ont des valeurs numériques qui varient de 0 à 10, et on fait réagir les composés (II) ensuite avec la N-acetylglycine, la N-acetylalanine, la N-acetylleucine ou avec les N-acetylpeptides,comportant de 2 à 5 aminoacides,qui sont liés peptidiques et éventuellement à l'aide d'une étape réactionelle supplémentaire on transforme les composés obtenus en leurs sels pharmaceutiquement acceptables.

2. Procédé suivant la revendication 1, caractérisé en ce que l'on utilise des acides nitratogras d'une longueur de chaînes de C₂ à C₆, qui sont linéaires ou ramifiés, qui sont des racémiques ou des isomères optiques.

3. Procédé suivant les revendications 1 et 2, caractérisé en ce que l'on utilise, à titre d'aminoacides, contenant de soufre, la cystéine, la méthionine, l'homocysteine ou la β, β-diméthylcysteine.

4. Procédé suivant l'une quelconque des revendications 1 à 3, caractérisé en ce que l'on utilise, à titre d'aminoacides, des acides de la forme stéréochimique L.

5. Procédé suivant l'une quelconque des revendications 1 à 4, caractérisé en ce que l'on utilise les aminoacides, contenant de soufre, estérifiés au bout C-terminal.

6. Procédé suivant l'une quelconque des revendications 1 à 5, caractérisé en ce que l'on utilise les aminoacides cystéine, homocystéine, β, β-diméthylcystéine et/ou méthionine sous la forme de leurs esters méthyliques, éthyliques ou propyliques.

7. Procédé de préparation de dérivés des nitrato acides alkanoiques ainsi que de leurs sels pharmaceutiquement acceptables selon revendication 1, caractérisé en ce que les composés obtenus repondent aux formes chimiques suivantes:
a)ester éthylique de la N-nitratopivaloyl-S-(N-acetylglycyl)-L-cystéine,
b)ester éthylique de la N-nitratopivaloyl,-S-(N-acetylalanyl)-L-cystéine,
c)ester éthylique de la N-nitratopivaloyl-S-(N-acetylleucyl)-L-cystéine.

8. Procédé de préparation de médicaments, caractérisé en ce que l'on transforme un composé ou un mélange de composés préparé selon des revendications 1 à 7 et/ou leurs dérivés et/ou leurs sels pharmaceutiquement acceptables, en une forme de médicament, éventuellement à l'aide d'adjuvants ou véhicules pharmaceutiques.
